(19) Europäisches Patentamt
European Patent Office
Office européen des brevets

(11) Publication number: **0 154 353 B1**

(12) # EUROPEAN PATENT SPECIFICATION

(45) Date of publication of patent specification: **15.11.89**

(51) Int. Cl.[4]: **C 07 D 207/08**, A 61 K 31/40

(21) Application number: **85102668.2**

(22) Date of filing: **08.03.85**

(54) **An anti-amnestic agent.**

(30) Priority: **09.03.84 JP 43751/84**

(43) Date of publication of application:
**11.09.85 Bulletin 85/37**

(45) Publication of the grant of the patent:
**15.11.89 Bulletin 89/46**

(84) Designated Contracting States:
**BE CH DE FR GB IT LI NL SE**

(56) References cited:
**CHEMICAL ABSTRACTS, vol. 99. no. 13, 26th September 1983, page 280, no. 101433t, Columbus, Ohio, US; S. WILK et al.: "Inhibition of rabbit brain prolyl endopeptidase by N-benzyloxycarbonyl-prolyl-prolinal, a transition state aldehyde inhibitor", & J. NEUROCHEM. 1983, 41(1), 69-75**
**CHEMICAL ABSTRACTS, vol. 100. no. 11, 12th March 1984, page 364, no. 83478u, Columbus, Ohio, US; T.C. FRIEDMAN et al.: "Prolyl endopeptidase: inhibition in vivo by N-benzyloxycarbonyl-prolyl-prolinal", & J. NEUROCHEM. 1984M 42(1), 237-41**

(73) Proprietor: **KABUSHIKI KAISHA YAKULT HONSHA**
**1-19, Higashishinbashi 1-chome**
**Minato-ku Tokyo 105 (JP)**

(72) Inventor: **Tsuru, Daisuke**
**10-16-502, Shiratorimachi**
**Nagasaki-shi Nagasaki-ken (JP)**
Inventor: **Yoshimoto, Tadashi**
**2-29-10, Nameishi**
**Nagasaki-shi Nagasaki-ken (JP)**
Inventor: **Hagiwara, Hisao**
**K.K. Yakult Honsha 1-19, Higashishinbashi 1-chome**
**Minato-ku Tokyo (JP)**
Inventor: **Kado, Kunio**
**K.K. Yakult Honsha 1-19, Higashishinbashi 1-chome**
**Minato-ku Tokyo (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 154 353 B1

(56) References cited:
CHEMICAL ABSTRACTS, vol. 101, no. 13, 24th September 1984, page 268, no. 106316b, Columbus, Ohio, US; H. YOKOSAWA et al.: "N-benzyloxycarbonyl-valyl-prolinal, a potent inhibitor of post-proline cleaving enzyme", J. BIOCHEM. (TOKYO), 1984, 95(6), 1819-21

CHEMICAL ABSTRACTS, vol. 95, no. 21, 23th November 1981, page 146, no. 198226f, Columbus, Ohio, US, A. CHERKIN et al.: "Amnestic effect of L-proline does not depend upon inhibition of brain protein synthesis", & BRAIN RES. 1981, 223(2), 455-8

CHEMICAL ABSTRACTS, vol. 93, no. 7, 18th August 1980, page 200, no. 62554n, Columbus, Ohio, US; A. VAN HARREVELD et al.: "Amnestic potency of proline analogs correlates with antispreading depression potency", & PHARMACOL. BIOCHEM. BEHAV. 1980, 12(4), 533-41

## Description

The present invention relates to a new type of anti-amnestic agent. More particularly, the present invention relates to a new type of anti-amnestic agent containing a specific prolinal derivative as an active ingredient thereof.

In recent years, it has become clear that vasopressin, oxytocin and the like peptide hormones participate in memory. It has been reported that the administration of such peptide hormones to experimentally induced amnestic rats resulted in a significant recovery of memory [Béla Bohus et al., Brain Research *157* (1978) 414—417]. It has also been reported that such peptide hormones showed a similar effect in clinical tests in humans [H. Weingartner et al., Science *211*, (1981), 601—603]. It is known that all of these peptide hormones contain proline and exist in the brain. These peptide hormones are thought to be metabolized by internal peptidase enzymes, especially a proline-specific endopeptidase (post-proline cleaving enzyme). Studies in the past have concerned proline-specific endopeptidase inhibitors. It has been found that N-benzyloxycarbonylglycyl-L-prolyl-chloromethane and N-benzyloxycarbonyl-L-prolyl prolinal possess a potent inhibitory action on the endopeptidase enzymes [T. Yoshimoto et al., Biochem. *16* (1977), 2942—2948 and S. Sherwin et al. J. Neurochem. *41* (1983), 69—75). Further investigations are required to study the biochemical activities of such endopeptidase inhibitors.

As a result of extensive research on endopeptidase inhibitors, it has been found unexpectedly that prolinal derivatives of the general formula I

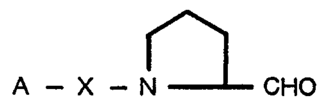

(I)

wherein A is a protective group for an amino group and X is a residue of an amino acid, possess potent anti-amnestic actions.

It is an object of the present invention to provide said prolinal derivatives of the general formula I for use as active therapeutic substances, in particular, for combating amnesia and memory disorders.

A further object of the previous invention is to provide novel prolinal derivatives of the general formula I wherein A is a protective group for an amino group and X is a residue of an amino acid with the proviso that A—X is not an N-benzyloxy prolyl or N-benzyloxyvalyl group.

Compounds wherein A—X is a N-benzyloxy-propyl or N-benzyloxy-valyl group are known from J. Neurochem., 1983, 41(1), p. 69—75, and J. Biochem. (Tokyo), 1984, 95 (6), p. 1819—1821.

The fact that a prolinal derivative represented by the above general formula exhibits a potent anti-amnestic activity is novel.

Investigations by the present inventors on the effect of prolinal derivatives of the general formula I on passive avoidance in rats, have shown that these prolinal derivatives administered exhibit extremely potent anti-amnestic activity (orally or intraperitoneally) and are not very toxic substances so that they can be safely utilized, for example, for the treatment of dementia senilis of the Alzheimer type.

The prolinal derivatives of the general formula I can be prepared according to conventional methods of amino acid chemistry. For example, an amino-protected amino acid is reacted with N-hydroxysuccinimide and a carbodiimide such as dicyclohexylcarbodiimide is added under cooling and then L-prolinal to form an oily or solid product which is then treated with 1-(3-dimethylaminopropyl)-3-ethyl carbodiimide. The protective group A for the amino group of an amino acid corresponding to X is known in the field of amino acid chemistry. Typical examples are the N-benzyloxycarbonyl, t-butyloxycarbonyl and a lower acyl group with 2—4 carbon atoms such as the acetyl group.

The prolinal derivatives of the general formula I can be administered to patients in various ways such as by an injection, for example, intravenous, subcutaneous or intramuscular injection and chewable or drinkable preparations suitable for oral administration. Normally, the prolinal derivatives are used singly or as a mixture of an least two. Further, the prolinal derivatives are normally blended with a suitable excipient generally used for pharmaceutical formulations. Suitable excipients include one or more organic and inorganic substances which are suitable for enteral or parenteral administration and do not react with the prolinal derivatives of the general formula I, for example, water, vegetable oils, benzyl alcohol, polyethylene glycols, gelatin, carbohydrates such as lactose and starch, magnesium stearate, talc or white petroleum jelly. Other organic and inorganic substances usually employed for preparing various medicaments can also be used as the excipient unless they influence the effect of the prolinal derivatives. The formulations used for oral administration must be easily absorbed in the digestive tract and may be, powders, tablets, pills, dragees, hard and soft capsules, syrups, juices, drops, elixirs and other orally acceptable liquid preparations, preferably oily or aqueous solutions, suspensions and emulsions. The formulations for various types of injections are as a rule in the form of the above liquid preparations. For injection, the prolinal derivatives may be lyophilized and the resulting lyophilizate may be used as needed for such injection preparations. Such formulations can be sterilized and/or contain one or more auxiliary substances such as lubricants, preservatives, stabilizers and/or wetting agents, emulsifiers, salts for influencing the osmotic pressure, buffer substances, dyestuffs, flavoring agents and/or aroma substances. They can also contain, if desired, one or more additional active compounds, for example, lecithin, one or more vitamins and depressants.

The particularly preferable modes of administration of the prolinal derivatives are oral administration and intravenous injection. The daily dose of the prolinal derivatives is preferably between 1 mg and 900 mg/kg, especially between 5 mg and 500 mg/kg of body weight for oral adminstration and between 0.5 mg and 500 mg/kg, especially between 1 mg and 200 mg/kg of body weight in case of intravenous injection. In the anti-amnestic agents of the pesent invention, the prolinal derivative or derivatives are contained generally at a concentration of 0.1% or more, preferably 1—50% by weight.

Described below are the anti-amnestic action and toxicity of the prolinal derivatives of the general formula I.

1. Anti-amnestic action

The anti-amnestic action was assayed according to the method reported by A. Kubota et al. in Int. Symp. on the Pharmacology of Learning and Memory, at Hakone, Japan (July 25, 1981) measuring the effect of the prolinal derivatives on passive avoidance responses in rats. The passive avoidance chamber used in this test was surrounded by one-way-visible plastic plates and included an electrified grid floor (30 cm in length and 30 cm in width) supplied with an electric current of 1.7 mA for electric foot shock and a platform (15 cm in length, 15 cm in width and 4 cm in height) placed at the right back corner thereof. Male Wistar SLC rats (weighing 100—150 g) were placed singly in the passive avoidance chamber and, on stepping down on the floor, a continuous electric current was sent to the grid until the rat went up on the platform. Rats staying on the platform for more than 15 seconds were taken out of the chamber, and were considered to have learned the situation. This electric shock had to normally be experienced 2 or 3 times. To avoid any difference in the rats, those requiring 10 seconds for the first step-down and those which stepped down on the floor at least 5 times were excluded. A control group of trained rats, were treated with intraperitoneal administration of 0.5% CNC solution while the other groups were treated with intraperitoneal administration of scopolamine hydrobromide at a dose of 3 mg/Kg to induce amnesia. The prolinal derivatives of the general formula to be tested were administered intraperitoneally or orally to the rats in an amount shown in Table 1 or 2 after completion of the learning process. In each case, the prolinal derivatives were dissolved or suspended in a 0.5% CMC solution. Results of the tests are shown in Tables 1 and 2. Intraperitoneal and oral administration of prolinal derivatives resulted in a significant extension of the latency time as compared to control values.

2. Toxicity test

Using 4-weeks-old male ICR mice weighing 15—20 g, the $LD_{50}$ value was calculated following oral or intraperitoneal administration of the prolinal derivatives. Results of these toxicity tests are shown in Table 3.

In view of the results shown in Tables 1—3, it is evident that the prolinal derivatives of the general formula I possess excellent anti-amnestic actions with slight toxicity and are thus utilizable in the treatment of memory disorders such as dementia senilis of the Alzheimer type. Throughout the specification the percentage is by weight unless otherwise indicated.

TABLE 1
Effects of various Prolinal Derivatives on Amnesia (i.p.)

| Agent used | Dosage (mg/kg) | Adminis-tration route | Treatment after Training | Training | | | Retention Test | | |
|---|---|---|---|---|---|---|---|---|---|
| | | | | Step down Latency (sec) | Number of descends Training during (times) | Total Time required for Test (sec) | Latency (sec) | | Number of Animals used |
| | | | | | | | 24 hr | 48 hr | |
| 0.5%CMC (Control) | — | i.p. | 0.5%CMC | 2.0 | 1.2 | 29.8 | 181.0 | 180.6 | 5 |
| 0.5%CMC (Control) | — | i.p. | Scopolamine | 2.4 | 1.8 | 30.6 | 117.4 | 106.2 | 5 |
| N-Benzyloxycarbonyl-glycyl-prolinal | 1.9 | i.p. | Scopolamine | 2.2 | 1.6 | 50.8 | >300.0 | >300.0 | 5 |
| N-Benzyloxycarbonyl-alanyl-prolinal | 2.0 | i.p. | Scopolamine | 1.8 | 1.0 | 35.8 | 189.0 | 203.4 | 5 |
| N-Benzyloxycarbonyl-valyl-prolinal | 2.2 | i.p. | Scopolamine | 1.2 | 2.4 | 28.2 | >300.0 | >300.0 | 5 |
| N-Benzyloxycarbonyl-prolyl-prolinal | 2.2 | i.p. | Scopolamine | 6.7 | 1.3 | 39.5 | 192.0 | 141.5 | 4 |
| N-Benzyloxycarbonyl-leucyl-prolinal | 3.5 | i.p. | Scopolamine | 2.3 | 1.4 | 38.5 | >300.0 | >300.0 | 5 |
| t-Butyloxycarbonyl-alanyl-prolinal | 1.8 | i.p. | Scopolamine | 1.9 | 1.5 | 28.5 | 293.5 | 250.4 | 5 |
| t-Butyloxycarbonyl-phenylalanyl-prolinal | 3.0 | i.p. | Scopolamine | 2.0 | 2.0 | 32.3 | >300.0 | 264.3 | 5 |
| t-Butyloxycarbonyl-seryl-prolinal | 2.9 | i.p. | Scopolamine | 1.8 | 1.9 | 39.6 | >300.0 | >300.0 | 5 |
| t-Butyloxycarbonyl-tyrosyl-prolinal | 3.6 | i.p. | Scopolamine | 2.2 | 1.4 | 34.7 | 250.8 | 206.9 | 4 |
| Acetyl-glycyl-prolinal | 1.3 | i.p. | Scopolamine | 2.3 | 1.4 | 30.0 | 274.9 | 198.6 | 5 |
| Acetyl-propyl-prolinal | 2.3 | i.p. | Scopalamine | 1.9 | 1.5 | 41.3 | >300.0 | >300.0 | 4 |

Remarks: Scopolamine (3 mg/kg) was administered intraperitoneally

TABLE 2

Effects of various Prolinal Derivatives on Amnesia (p.o.)

| Agent used | Dosage (mg/kg) | Adminis-tration route | Treatment after Training | Training | | | Retention Test | | |
|---|---|---|---|---|---|---|---|---|---|
| | | | | Step down Latency (sec) | Number of descends during Training (times) | Total Time required for Test (sec) | Latency (sec) 24 hr | Latency (sec) 48 hr | Number of Animals used |
| 0.5%CMC (Control) | — | p.o. | 0.5%CMC | 2.2 | 1.7 | 29.7 | 260.7 | 204.8 | 5 |
| 0.5%CMC (Control) | — | p.o. | Scopolamine | 6.9 | 2.6 | 52.4 | 121.7 | 80.5 | 6 |
| N-Benzyloxycarbonyl-glycyl-prolinal | 1.9 | p.o. | Scopolamine | 4.0 | 2.5 | 49.2 | 230.4 | 175.5 | 5 |
| N-Benzyloxycarbonyl-alanyl-prolinal | 2.0 | p.o. | Scopolamine | 3.5 | 1.8 | 36.5 | 165.4 | 155.2 | 5 |
| N-Benzyloxycarbonyl-valyl-prolinal | 2.2 | p.o. | Scopolamine | 2.8 | 2.4 | 32.0 | 254.8 | 210.5 | 6 |
| N-Benzyloxycarbonyl-prolyl-prolinal | 2.2 | p.o. | Scopolamine | 3.9 | 1.3 | 36.5 | 140.2 | 102.3 | 5 |
| N-Benzyloxycarbonyl-leucyl-prolinal | 3.5 | p.o. | Scopolamine | 2.3 | 1.6 | 32.1 | 281.6 | 219.5 | 5 |
| t-Butyloxycarbonyl-alanyl-prolinal | 1.8 | p.o. | Scopolamine | 4.3 | 2.1 | 52.4 | 239.8 | 226.7 | 5 |
| t-Butyloxycarbonyl-phenylalanyl-prolinal | 3.0 | p.o. | Scopolamine | 5.6 | 2.0 | 47.1 | >300.0 | 250.2 | 4 |
| t-Butyloxycarbonyl-seryl-prolinal | 2.9 | p.o. | Scopolamine | 2.9 | 1.9 | 45.2 | 262.9 | 185.2 | 5 |
| t-Butyloxycarbonyl-tyrosyl-prolinal | 3.6 | p.o. | Scopolamine | 4.3 | 1.8 | 32.5 | 180.5 | 192.1 | 5 |

TABLE 2 (continued)

| Agent used | Dosage (mg/kg) | Adminis-tration route | Treatment after Training | Training | | | Retention Test | | |
|---|---|---|---|---|---|---|---|---|---|
| | | | | Step down Latency (sec) | Number of descends during Training (times) | Total Time required for Test (sec) | Latency (sec) | | Number of Animals used |
| | | | | | | | 24 hr | 48 hr | |
| Acetyl-glycyl-prolinal | 1.3 | p.o. | Scopolamine | 3.3 | 1.5 | 35.5 | 221.6 | 143.2 | 5 |
| Acetyl-prolyl-prolinal | 2.3 | p.o. | Scopolamine | 1.9 | 1.4 | 40.3 | 204.2 | 177.4 | 5 |

Remarks: Scopolamine (3 mg/kg) was administered intraperitoneally

TABLE 3
$LD_{50}$ (ip) Values of Various Prolinal Derivatives

| Prolinal Derivatives | $LD_{50}$ (mg/kg) |
|---|---|
| N-Benzyloxycarbonyl-glycyl-prolinal | >1,000 |
| N-Benzyloxycarbonyl-alanyl-prolinal | 954 |
| N-Benzyloxycarbonyl-valyl-prolinal | 825 |
| N-Benzyloxycarbonyl-prolyl-prolinal | >1,000 |
| N-Benzyloxycarbonyl-leucyl-prolinal | >1,000 |
| t-Butyloxycarbonyl-alanyl-prolinal | 796 |
| t-Butyloxycarbonyl-phenylalanyl-prolinal | >1,000 |
| t-Butyloxycarbonyl-seryl-prolinal | >1,000 |
| t-Butyloxycarbonyl-tyrosyl-prolinal | 933 |
| Acetyl-glycyl-prolinal | >1,000 |
| Acetyl-prolyl-prolinal | 767 |

Animals used: ICR male mice

The following examples illustrate the preparation of the various prolinal derivatives of the general formula I used as the effective ingredient in the anti-amnestic agents of the present invention wherein all the amino acids except glycin were used in L-form. In these Examples the abbreviations used for the amino acid residues are as follows:

| | |
|---|---|
| Ala : alanine | Val : valine |
| Gly : glycin | Leu : leucine |
| Phe : phenylalanine | Ser : serine |
| Pro : proline | Tyr : tyrosine |

The abbreviations used for the compounds and for the protecting groups of the amino group of the amino acid structures are as follows:

| | | |
|---|---|---|
| DCC | : | dichlorohexylcarbodiimide |
| WSCD·HCl | : | 1-(3-dimethylaminopropyl)-3-ethylcarbodiimide hydrochloride |
| HOSu | : | N-hydroxysuccinimide |
| THF | : | tetrahydrofuran |
| DMSO | : | dimethylsulfoxide |
| DMF | : | dimethylformamide |
| Z | : | benzyloxycarbonyl group |
| t-Boc | : | butyloxycarbonyl |
| Ac | : | acetyl. |

Example 1

Preparation of N-benzyloxycarbonyl-alanyl-prolinal of the formula (1)

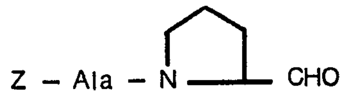

(1)

22.3 g of N-Benzyloxycarbonylalanine were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated to dryness whereby a semi-solid substance was obtained. This substance was recrystallized from isopropyl alcohol to obtain a white solid substance which was then dissovled in 470 ml of THF. To this solution 7.4 g of L-prolinol were added and the mixture was stirred for 72 hours at room temperature. After removal of THF by distillation, the residue was dissolved in 300 ml of ethyl acetate and the solution was washed with 100 ml of 1—M $Na_2CO_3$ solution, dried over anhydrous $Na_2SO_4$ and evaporated to dryness. To the resultant oily substance 32.4 g of WSCD·HCl and 180 ml of redistilled DMSO were added and the mixture was stirred for 10 minutes. Subsequently 6 ml of a 2M DMSO solution of anhydrous $H_3PO_4$ were added and the mixture was stirred for further 2 hours. The reaction was stopped with 360 ml of a 1 H buffer solution of potassium phosphate of pH 7.5. The reaction mixture was extracted with 250 ml of ethyl acetate, and the extract was dried over anhydrous $Na_2SO_4$. The ethyl acetate was distilled off and the residue was dissolved in 250 ml of ethanol. To this solution 200 g of $NaHSO_3$ in 350 ml of water were added and the mixture was stirred vigorously for 10 minutes. After concentration of the ethanol to about 350 ml, unreacted materials were removed using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 2.9 g (9.9%).

| | C | H | N |
|---|---|---|---|
| $C_{16}H_{20}N_2O_4$; calc. | 63.14 | 6.62 | 9.20 |
| found | 63.10 | 6.49 | 9.29 |

Example 2

Preparation of N-benzyloxycarbonyl-glycyl-prolinal of the Formula (2)

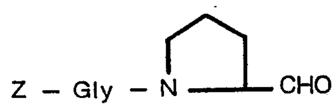

(2)

17.7 g of N-Benzyloxycarbonyl-glycin were dissolved in 200 ml of THF. During cooling using NaCl and ice 11.5 g of HOSu and 20.6 g of DCC were added and the mixture was stirred for 21 hours at 4°C. The reaction liquid was filterd off and the filtrate was evaporated whereby a semi-solid substance was obtained. This substance was recrystallized from isopropyl alcohol to obtain a white solid substance which was then dissolved in 600 ml of THF. To this solution 9.2 g of L-prolinol were added and the mixture was stirred for 72 hours at room temperature. After removal of THF by distillation, the residue was dissolved in 300 ml of ethyl acetate and the solution was washed with 100 ml of a 1M $Na_2CO_3$ solution, dried with anhydrous $Na_2SO_4$ and evaporated to dryness. To the resultant oily substance 38.7 g of WSCD·HCl and 155 ml of redistilled DMSO were added and the mixture was stirred for 10 minutes. Subsequently, 7.7 ml of a 2M DMSO solution of anhydrous $H_3PO_4$ were added and the mixture was stirred for further 2 hours. The reaction was stopped with 360 ml of a 1M buffer solution of potassium phosphate of pH 7.5. The reaction mixture was extracted with 250 ml of ethyl acetate and the extract was dried over anhydrous $Na_2SO_4$. The ethyl acetate was distilled off and the residue was dissolved in 300 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 4.5 g (15.6%).

Example 3

Preparation of N-benzyloxycarbonyl-valyl-prolinal of the formula (3)

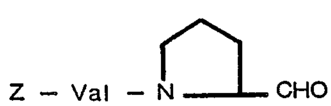

(3)

22.1 g of N-Benzyloxycarbonylvaline were dissolved in 200 ml of THF. During cooling using NaCl and ice 11.5 g of HOSu and 20.6 g of DCC were added and the mixture was stirred for 21 hours at 4°C. The reaction

liquid was filtered off and the filtrate was evaporated whereby a semi-solid substance was obtained. This substance was recrystallized from isopropyl alcohol to obtain a white solid substance which was then dissolved in 470 ml of THF. To this solution 8.6 g of L-prolinol were added and the mixture was stirred for 72 hours at room temperature. After removal of THF by distillation, the residue was dissolved in 300 ml of ethyl acetate and the solution was washed with 100 ml of 1M $Na_2CO_3$, dried with anhydrous $Na_2SO_4$ and evaporated to dryness. To the resultant oily substance 43.1 g of WSCD·HCl and 150 ml of redistilled DMSO were added and the mixture was stirred for 10 minutes. Subsequently, 7 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 200 ml of ethyl acetate and the extract was dried over anhydrous $Na_2SO_4$. The ethyl acetate was distilled off and the residue was dissolved in 200 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ were added until the pH value of the liquid became 9. After 5 minutes, the reaction liquid was extracted twice with 100 ml of ether. The ether was then distilled off to leave an oily substance. Yield 5.7 g (17.2%).

| | C | H | N |
|---|---|---|---|
| $C_{18}H_{24}N_2O_4$; calc. | 65.04 | 7.28 | 8.43 |
| found | 64.82 | 7.39 | 8.39 |

Example 4

Preparation of N-benzyloxycarbonyl-prolyl-prolinal of the formula (4):

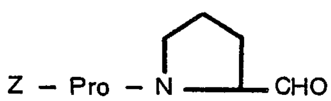

(4)

22.1 g of N-Benzyloxycarbonyl-proline were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated whereby a semi-solid substance was obtained. This substance was recrystallized from isopropyl alcohol to obtain a white solid substance which was then dissolved in 450 ml of THF. To this solution 8.2 g of L-prolinol were added and the mixture was stirred for 72 hours at room temperature. After removal of THF by distillation, the residue was dissolved in 300 ml of ethyl acetate and the solution was washed with 100 ml of 1M $Na_2CO_3$, dried with anhydrous $Na_2SO_4$ and evaporated to dryness. To the resultant white solid substance 40.5 g of WSCD·HCl and 150 ml of redistilled DMSO were added and the mixture was stirred for 10 minutes. Subsequently, 6.3 ml of a 2M DMSO solution of anhydrous $H_3PO_4$ were added and the mixture was stirred for further 2 hours. The reaction was stopped with 350 ml of a 1M buffer solution of potassium phosphate of pH 7.5. The reaction mixture was extracted with 200 ml of ethyl acetate, and the extract was dried over anhydrous $Na_2SO_4$. The ethyl acetate was distilled off and the residue was dissolved in 200 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 3.4 g (10.3%).

| | C | H | N |
|---|---|---|---|
| $C_{18}H_{22}N_2O_4$; calc. | 65.44 | 6.70 | 8.48 |
| found | 65.28 | 6.83 | 8.59 |

$IR\nu_{max}^{KBr}cm^{-1}$: 2995, 2880, 1690, 1645, 1420, 1355, 1045.

Example 5

Preparation of N-benzyloxycarbonyl-leucyl-prolinal of the formula (5):

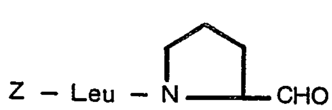

(5)

26.5 g of N-Benzyloxycarbonyl-leucin were dissolved in 200 ml of THF. During cooling using NaCl and ice 11.5 g of HOSu and 20.6 g of DCC were added, and the mixture was stirred for 21 hours at 4°C. The reaction liquid was filtered off and the filtrate was evaporated whereby a semi-solid substance was obtained. This substance was recrystallized from isopropyl alcohol to obtain a white solid substance which was then dissolved in 400 ml of THF. To this solution 7.9 g of L-prolinol were added and the mixture was stirred for 72 hours at room temperature. After removal of THF by distillation, the residue was dissolved in 300 ml of

ethyl acetate and the solution was washed with 100 ml of 1M $Na_2CO_3$, dried with anhydrous $Na_2SO_4$ and evaporated to dryness. To the resultant oily substance 33.5 g of WSCD·HCl and 140 ml of redistilled DMSO were added and the mixture was stirred for 10 minutes. Subsequently, 6.2 ml of 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 300 ml of ethyl acetate, and the extract was dried over anhydrous $Na_2SO_4$. The ethyl acetate was distilled off and the residue was dissolved in 300 ml of ethanol. To this solution 200 g of $NaHSO_3$ in 350 ml of water were added, and the mixture was stirred vigorously for 10 minutes. After concentration of the ethanol up to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 4.2 g (12.1%).

Example 6

Preparation of t-butyloxycarbonyl-alanyl-prolinal of the formula (6):

t–Boc – Ala – 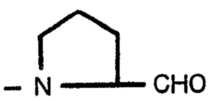 (6)

2.1 g of t-Butyloxycarbonylalanine were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated whereby a semi-solid substance was obtained. This substance was recrystallized from chloroform to obtain a white solid substance which was then dissoved in 400 ml of THF. To this solution 7.5 g of L-prolinol were added and the mixture was stirred for 72 hours at room temperature. After removal of THF by distillation, the residue was dissolved in 300 ml of ethyl acetate and the solution was washed with 100 ml of 1M $Na_2CO_3$, dried with anhydrous $Na_2SO_4$ and evaporated to dryness. To the resultant oily substance 32.5 g of WSCD·HCl and 130 ml of redistilled DMSO were added and the mixture was stirred for 10 minutes. Subsequently, 6 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 250 ml of ethyl acetate and the extract was dried over anhydrous $Na_2SO_4$. The ethyl acetate was distilled off and the residue was dissolved in 250 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 1.2 g (4.5%).

| | C | H | N |
|---|---|---|---|
| $C_{13}H_{22}O_4N_2$; calc. | 57.76 | 8.20 | 10.36 |
| found | 57.81 | 8.30 | 10.22 |

Example 7

Preparation of t-butyloxycarbonyl-phenylalanyl-prolinal of the formula (7):

t–Boc – Phe – 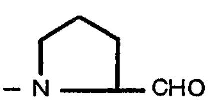 (7)

29.9 g of t-Butyloxycarbonyl-phenylalanine were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated whereby a semi-solid substance was obtained. This substance was recrystallized from chloroform to obtain a white solid substance which was then dissolved in 400 ml of THF. To this solution 7.4 g of L-prolinol were added and the mixture was stirred for 72 hours at room temperature. After removal of THF by distillation, the residue was dissolved in 300 ml of ethyl acetate and the solution was washed with 100 ml of 1M $Na_2CO_3$, dried with anhydrous $Na_2SO_4$ and evaporated to dryness. To the resultant oily substance 29.2 g of WSCD·HCl and 120 ml of redistilled DMSO were added and the mixture was stirred for 10 minutes. Subsequently, 5.8 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 250 ml of ethyl acetate. The extract was dried over anhydrous $Na_2SO_4$. The ethyl acetate was distilled off and the residue was dissolved in 250 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 3.3 g (8.4%).

Example 8

Preparation of t-butyloxycarbonyl-seryl-prolinal of the formula (8):

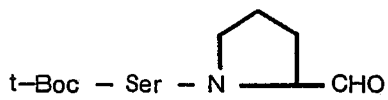

(8)

20.5 g of t-Butyloxycarbonylserine were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated whereby a semi-solid substance was obtained. This substance was recrystallized from chloroform to obtain a white solid substance which was then dissolved in 450 ml of THF. To this solution 18.0 g of L-prolinol were added and the mixture was stirred for 72 hours at room temperature. After removal of THF by distillation, the residue was dissolved in 330 ml of ethyl acetate and the solution was washed with 100 ml of 1M $Na_2CO_3$, dried with anhydrous $Na_2SO_4$ and evaporated to dryness. To the resultant oily substance 36.9 g of WSCD·HCl and 150 ml of redistilled DMSO were added and the mixture was stirred for 10 minutes. Subsequently, 6.5 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 300 ml of ethyl acetate and the extract was dried over anhydrous $Na_2SO_4$. The ethyl acetate was distilled off and the residue was dissolved in 250 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 2.1 g (7.3%).

Example 9

Preparation of t-butyloxycarbonyl-tyrosyl-prolinal of the formula (9):

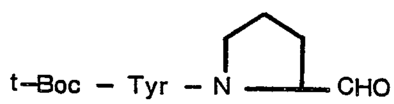

(9)

28.1 g of t-Butyloxycarbonyltyrosine were dissolved in 200 ml of THF. During cooling using NaCl and ice 11.5 g of HOSu and 20.6 g of DCC were added and the mixture was stirred for 21 hours at 4°C. The reaction liquid was filtered off and the filtrate was evaporated whereby a semi-solid substance was obtained. This substance was recrystallized from chloroform to obtain a white solid substance which was then dissolved in 500 ml of THF. To this solution 6.0 g of L-prolinol were added and the mixture was stirred for 72 hours at room temperature. After removal of THF by distillation, the residue was dissolved in 200 ml of ethyl acetate and the solution was washed with 100 ml of 1M $Na_2CO_3$, dried with anhydrous $Na_2SO_4$ and evaporated to dryness. To the resultant oily substance 20.3 g of WSCD·HCl and 90 ml of redistilled DMSO were added and the mixture was stirred for 10 minutes. Subsequently, 3.9 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 200 ml of ethyl acetate, and the extract was dried over anhydrous $Na_2SO_4$. The ethyl acetate was distilled off and the residue was dissolved in 200 ml of ethanol. To this solution 200 g of $NaHSO_3$ in 350 ml of water were added and the mixture was stirred vigorously for 10 minutes. After concentration of the ethanol to about 350 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 0.8 g (2.2%).

Example 10

Preparation of acetyl-glycyl-prolinal of the formula (10):

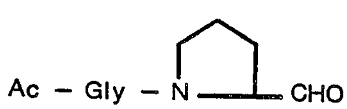

(10)

11.7 g of Acetyl-glycin were dissolved in 150 ml of THF. To this solution 11.5 g of HOSu and 20.6 g of DCC were added and the mixture was stirred for 21 hours at 4°C. The reaction liquid was filtered off and the filtrate was evaporated under reduced pressure whereby a semi-solid substance was obtained. This substance was recrystallized from chloroform to obtain a white solid substance which was then dissolved in 350 ml of THF. To this solution 4.2 g of L-prolinol were added and the mixture was stirred for 72 hours at room temperature. After removal of THF by distillation, the residue was dissolved in 200 ml of ethyl acetate and the solution was washed with 100 ml of 1M $Na_2CO_3$, dried with anhydrous $Na_2SO_4$ and evaporated to dryness. To the resultant oily substance 19.6 g of WSCD·HCl and 75 ml of redistilled DMSO were added and

the mixture was stirred for 10 minutes. Subsequently, 3.2 ml of a 2M DMSO solution of anhydrous $H_3PO_4$ were added and the mixture was stirred for further 2 hours. The reaction was stopped with 160 ml of a 1M buffer solution of potassium phosphate of pH 7.5. The reaction mixture was extracted with 150 ml of ethyl acetate and the extract was dried over anhydrous $Na_2SO_4$. The ethyl acetate was distilled off and the residue was dissolved in 100 ml of ethanol. To this solution 50 g of $NaHSO_3$ in 90 ml of water were added and the mixture was stirred vigorously for 10 minutes. After concentration of the ethanol to about 90 ml, unreacted materials were eliminated using 2 × 60 ml of ether. Solid $Na_2CO_3$ was added until the pH value of the liquid became 9. After 5 minutes, the reaction liquid was extracted twice with 60 ml of ether. The ether was then distilled off to leave an oily substance. Yield 0.3 g (1.5%).

Example 11

Preparation of acetyl-propyl-prolinal of the formula (11):

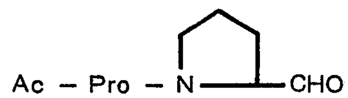

(11)

15.7 g of Acetyl-proline were dissolved in 200 ml of THF. During cooling using NaCl and ice 11.5 g of HOSu and 20.6 g of DCC were added, and the mixture was stirred for 21 hours at 4°C. The reaction liquid was filtered and the filtrate was evaporated whereby a semi-solid substance was obtained. This substance was recrystallized from isopropyl alcohol to obtain a white solid substance which was then dissoved in 400 ml of THF. To this solution 7.5 g of L-prolinol were added and the mixture was stirred for 72 hours at room temperature. After removal of THF by distillation, the residue was dissolved in 300 ml of ethyl acetate and the solution was washed with 100 ml of 1M $Na_2CO_3$, dried with anhydrous $Na_2CO_4$ and evaporated to dryness. To the resultant oily substance 35.8 g of WSCD·HCl and 160 ml of redistilled DMSO were added and the mixture was stirred for 10 minutes. Subsequently, 7.1 ml of a 2M DMSO solution of anhydrous $H_3PO_4$ were added and the mixture was stirred for further 2 hours. The reaction was stopped with 300 ml of a 1M buffer solution of potassium phosphate of pH 7.5. The reaction mixture was extracted with 350 ml of ethyl acetate, and the extract was dried over anhydrous $Na_2SO_4$. The ethyl acetate was distilled off and the residue was dissolved in 300 ml of ethanol. To this solution 200 g of $NaHSO_3$ in 350 ml of water were added and the mixture was stirred vigorously for 10 minutes. After concentration of the ethanol to about 300 ml, unreacted materials were eliminated using 2 × 100 ml of ether. Solid $Na_2CO_3$ was added until the pH value of the liquid became 9. After 5 minutes, the reaction liquid was extracted twice with 100 ml of ether. The ether was then distilled off to leave an oily substance. Yield 5.5 g (23.5%).

| | C | H | N |
|---|---|---|---|
| $C_{12}H_{19}N_2O_3$; calc. | 56.44 | 7.50 | 10.97 |
| found | 56.28 | 7.54 | 10.86 |

The following examples illustrate the preparation of the anti-amnestic agents of the present invention.

Example A Injection preparation

(1) Recipe

| | | |
|---|---|---|
| N-Benzyloxycarbonyl-glycyl-L-prolinal | | 10 mg |
| Hardened castor oil polyoxyethylene (60 mol) ether | | 40 mg |
| sorbitan monostearate | | 2 mg |
| Propylene glycol | | 60 mg |
| Refined soybean lecitin | | 2 mg |
| Cholesterol | | 1 mg |
| Dextrose | | 50 mg |
| Distilled water | to make | 1 ml |

(2) Preparation

N-Benzyloxycarbonyl-glycyl-L-prolinal, hardened castor oil polyoxyethylene (60 mol) ether, sorbitan monostearate, propylene glycol, refined soybean lecitin and chlolesterol are mixed and heated in a water bath at about 80°C to form a homogeneous liquid. To this liquid distilled water at about 80°C is added, with stirring to form a solubilized homogeneous system. Dextrose is then added. Distilled water is added to a

volume of 1 ml. The liquid is subjected to sterilizing filtration and filled into a brown ampoule which is then sealed.

Example B Soft capsulated preparation

(1) Recipe

| | |
|---|---|
| t-Butyloxycarbonyl-L-alanyl-prolinal | 20 mg |
| Macrogol 400 | 350 mg |
| Propylene glycol | 38 mg |
| Dispotassium glycyrrhizinate | 1 mg |
| Menthol oil | 1 mg |
| Gelatin | 122 mg |
| Glycerol | 30.5 mg |
| D-Sorbitol liquid | 12.2 mg |
| Ethyl p-hydroxybenzoate | 0.8 mg |
| Propyl p-hydroxybenzoate | 0.5 mg |

(2) Preparation

t-Butyloxycarbonyl-L-alanyl-prolinal, Macrogol 400, dipotassium glycyrrhizinate, menthol oil and propylene glycol are homogeneously blended to form a suspension. Separately, a coating agent for soft capsules is manufactured from gelatin, glycerol, D-sorbitol liquid, ethyl p-hydroxybenzoate and propyl p-hydroxybenzoate. Using the suspension and the coating agent, a soft capsule is prepared.

**Claims**

1. Prolinal derivatives of the general formula I

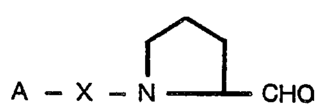

(I)

wherein A is a protective group for an amino group and X is a residue of an amino acid for use as active therapeutic substances.

2. Prolinal derivatives as claimed in claim 1 for use as active therapeutic substances combating amnesia and memory disorders.

3. Prolinal derivatives as claimed in claim 1 or 2, wherein the protective group A is an N-benzyloxycarbonyl group, a t-butyloxycarbonyl group or an acyl group containing 2 to 4 carbon atoms.

4. Prolinal derivatives of the general formula I

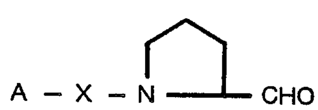

(I)

wherein A is a protective group for an amino group and X is a residue of an amino acid with the proviso that A—X is not a N-benzyloxy prolyl or N-benzyloxy valyl group.

5. Prolinal derivatives as claimed in claim 4, wherein A is an N-benzyloxycarbonyl group, a t-butyloxycarbonyl group or an acyl group containing 2 to 4 carbon atoms and X is an alanyl, glycyl, phenylalanyl, leucyl, seryl or tyrosyl group.

**Patentansprüche**

1. Prolinal-Derivate der allgemeinen Formel I

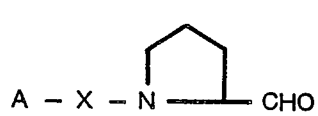

(I)

in der A eine Schutzgruppe für eine Aminogruppe bedeutet und X einen Rest einer Aminosäure darstellt, zur Verwendung als therapeutische Wirkstoffe.

2. Prolinal-Derivate nach Anspruch 1 zur Verwendung als therapeutisch Wirkstoffe zur Bekämpfung von Amnesie und Gedächtnisstörungen.

3. Prolinal-Derivate nach Anspruch 1 oder 2, wobei die Schutzgruppe A eine N-Benzyloxycarbonylgruppe, eine tert.-Butyloxycarbonylgruppe oder eine Acylgruppe mit 2 bis 4 Kohlenstoffatomen ist.

4. Prolinal-Derivate der allgemeinen Formel I

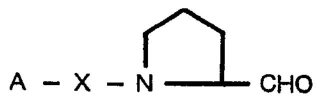

(I)

in der A eine Schutzgruppe für eine Aminogruppe bedeutet und X einen Rest einer Aminosäure darstellt, mit der Maßgabe, daß A—X nicht eine N-Benzyloxy Prolyl- oder N-Benzyloxy Valylgruppe darstellt.

5. Prolinal-Derivate nach Anspruch 4, in der A eine N-Benzyloxycarbonylgruppe, eine tert.-Butyloxycarbonylgruppe oder eine Acylgruppe mit 2 bis 4 Kohlenstoffatomen bedeutet und X eine Alanyl-, Glycyl-, Phenylalanyl-, Leucyl-, Seryl- oder Tyrosylgruppe darstellt.

## Revendications

1. Dérivés de prolinal de la formule générale I:

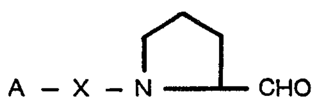

(I)

dans laquelle A représente un groupe de protection pour un groupe amino et X représente un reste d'un aminoacide, utilisables comme substances thérapeutiques actives.

2. Dérivés de prolinal suivant la revendication 1, utilisables comme substances thérapeutiques actives, combattant l'amnésie et les troubles de la mémoire.

3. Dérivés de prolinal suivant l'une ou l'autre des revendications 1 et 2, caractérisé en ce que le groupe de protection A est un groupe N-benzyloxycarbonyle, un groupe t-butyloxycarbonyle ou un groupe acyle contenant 2 à 4 atomes de carbone.

4. Dérivés de prolinal de la formule générale I:

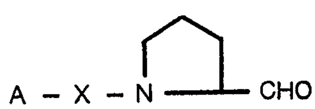

(I)

dans laquelle A représente un groupe de protection pour un groupe amino et X représente un reste d'un aminoacide pour autant que A—X ne représente pas un groupe N-benzyloxyprolyle ou N-benzyloxyvalyle.

5. Dérivés de prolinal suivant la revendication 4, caractérisés en ce que A représente un groupe N-benzyloxycarbonyle, un groupe t-butyloxycarbonyle ou un groupe acyle contenant 2 à 4 atomes de carbone et X représente un groupe alanyle, glycyle, phénylalanyle, leucyle, séryle ou tyrosyle.